(19) Europäisches Patentamt
European Patent Office
Office européen des brevets

(11) **EP 1 333 922 B1**

(12) **EUROPÄISCHE PATENTSCHRIFT**

(45) Veröffentlichungstag und Bekanntmachung des Hinweises auf die Patenterteilung:
**02.06.2004 Patentblatt 2004/23**

(51) Int Cl.[7]: **B01J 37/02**, B01J 23/887, C07C 45/35, C07C 47/22

(21) Anmeldenummer: **01986623.5**

(22) Anmeldetag: **09.10.2001**

(86) Internationale Anmeldenummer:
**PCT/EP2001/011643**

(87) Internationale Veröffentlichungsnummer:
**WO 2002/030569 (18.04.2002 Gazette 2002/16)**

(54) **VERFAHREN ZUR HERSTELLUNG EINES RINGFÖRMIGEN SCHALENKATALYSATORS UND VERWENDUNG DAVON ZUR HERSTELLUNG VON ACROLEIN**

METHOD FOR PRODUCING AN ANNULAR SHELL CATALYST AND USE THEREOF FOR PRODUCING ACROLEIN

PROCEDE DE PREPARATION D'UN CATALYSEUR A ENVELOPPE ANNULAIRE ET SON UTILISATION POUR PRODUIRE DE L'ACROLEINE

(84) Benannte Vertragsstaaten:
**AT BE CH CY DE DK ES FI FR GB GR IE IT LI LU MC NL PT SE TR**

(30) Priorität: **10.10.2000 DE 10049873**
**01.12.2000 DE 10059713**

(43) Veröffentlichungstag der Anmeldung:
**13.08.2003 Patentblatt 2003/33**

(73) Patentinhaber: **BASF AKTIENGESELLSCHAFT**
**67056 Ludwigshafen (DE)**

(72) Erfinder:
• **PETZOLDT, Jochen**
  **68163 Mannheim (DE)**
• **UNVERRICHT, Signe**
  **68169 Mannheim (DE)**
• **ARNOLD, Heiko**
  **210014 Nanjing (CN)**

(56) Entgegenhaltungen:
WO-A-00/53557        DE-A- 2 909 671
DE-A- 4 442 346      DE-A- 19 855 913

**Beschreibung**

Vorliegende Erfindung betrifft ein Verfahren zur Herstellung eines Katalysators, der aus einem Trägerkörper und einer auf der Oberfläche des Trägerkörpers aufgebrachten katalytisch aktiven Oxidmasse der allgemeinen Formel I

$$Mo_{12}Bi_aX^1_bFe_cX^2_dX^3_eO_y \qquad\qquad (I),$$

mit

X$^1$= Co und/oder Ni,
X$^2$= Si und/oder Al,
X$^3$= Li, Na, K, Cs und/oder Rb,
$0,2 \le a \le 1$,
$2 \le b \le 10$,
$0,5 \le c \le 10$,
$0 \le d \le 10$,
$0 \le e \le 0,5$ und
y= eine Zahl, die unter der Voraussetzung der Ladungsneutralität durch die Wertigkeit und Häufigkeit der von Sauerstoff verschiedenen Elemente in I bestimmt wird,

besteht,
bei dem man aus Ausgangsverbindungen der elementaren Konstituenten der katalytisch aktiven Oxidmasse ein inniges Trockengemisch erzeugt, das innige Trockengemisch bei einer Temperatur von 150 bis 350°C unter Erhalt einer Vorläufermasse thermisch behandelt, den Trägerkörper mit Wasser befeuchtet, danach durch Inkontaktbringen mit feinteiliger Vorläufermasse an der Oberfläche des befeuchteten Trägerkörpers eine Schicht der Vorläufermasse anheftet, anschließend den beschichteten Trägerkörper trocknet und abschließend den getrockneten, mit Vorläufermasse beschichteten, Trägerkörper bei einer Temperatur von 400 bis 600°C calciniert.

Ferner betrifft vorliegende Erfindung Katalysatoren, die nach vorstehenden Verfahren erhältlich sind und ihre Verwendung für die gasphasenkatalytische Oxidation von Propen zu Acrolein.

Die Herstellung des bedeutenden Zwischenproduktes Acrolein durch heterogen katalysierte Gasphasenoxidation von Propen ist allgemein bekannt (vgl. z.B. DE-A 19855913).

Verwendung findet Acrolein u.a. zur Herstellung von Acrylsäure, deren Alkylester insbesondere als Monomere zur Herstellung wäßriger Polymerdispersionen eingesetzt werden.

Es ist auch bekannt, als Katalysatoren für die heterogen katalysierte Gasphasenoxidation von Propen zu Acrolein als katalytisch aktive Oxidmassen Multimetalloxidmassen der allgemeinen Formel I einzusetzen.

Beispielsweise empfiehlt die DE-A 19855913 die Verwendung von ausschließlich aus aktiven Multimetalloxiden I geformten Ringen (Vollkatalysator-Ringe) sowie die Verwendung von auf kugelförmige Trägerkörper aufgebrachte aktive Multimetalloxide I (kugelförmige Schalenkatalysatoren) als Katalysatoren für die gasphasenkatalytisch oxidative Herstellung des Acroleins aus Propen.

Nachteilig an der in der DE-A 19855913 empfohlenen Verfahrensweise ist jedoch, daß einerseits die mechanische Stabilität der Vollkatalysator-Ringe und andererseits die Selektivität und Aktivität der Acroleinbildung bei den kugelförmigen Schalenkatalysatoren nicht voll zu befriedigen vermag. Das gleiche gilt für die kugelförmigen Schalenkatalysatoren der WO 95/26820.

Die DE-A 4442346 betrifft die Herstellung von ringförmigen Schalenkatalysatoren von aktiven Multimetalloxidmassen der allgemeinen Formel I. Als flüssiges Bindemittel beim Aufbringen der aktiven Multimetalloxide I empfiehlt die DE-A 4442346 die Verwendung einer Lösung, die aus 20 bis 90 Gew.-% Wasser und 10 bis 80 Gew.-% einer in Wasser gelösten organischen Verbindung besteht. Nachteilig an den ringförmigen Schalenkatalysatoren der DE-A 4442346 ist, daß auch ihre Aktivität und Selektivität bezüglich der Acroleinbildung bei Verwendung als Katalysatoren für die gasphasenkatalytische Partialoxidation von Propen nicht zu befriedigen vermag.

Die EP-A 900774 lehrt ebenfalls die Herstellung von Schalenkatalysatoren deren Aktivmassenstöchiometrie der allgemeinen Formel I entspricht.

Gemäß der Beschreibung der EP-A 900774 kann die Geometrie des zur Herstellung der Schalenkatalysatoren verwendeten Trägers beliebig sein. Weiterhin kommen gemäß der Beschreibung der EP-A 900774 als flüssige Bindemittel solche der unterschiedlichsten Art (einschließlich Wasser) in Betracht. In den Ausführungsbeispielen der EP-A 900774 werden ausschließlich kugelförmige Trägerkörper verwendet und als Bindemittel wird stets eine wäßrige Glycerinlösung angewandt.

Die in der EP-A 900774 hergestellten Schalenkatalysatoren werden zwar als Katalysatoren für die gasphasenkatalytische Oxidation von Propen zu Acrolein empfohlen, doch vermögen sie weder in Bezug auf ihre Aktivität noch in Bezug auf die Selektivität der Acroleinbildung zu befriedigen.

In der älteren Anmeldung DE-A 19948523 werden ebenfalls ringförmige Schalenkatalysatoren für die gasphasenkatalytisch Oxidation von Propen zu Acrylsäure empfohlen, doch enthält die DE-A 19948523 keine konkrete Verfahren zu deren Herstellung.

Die Aufgabe der vorliegenden Erfindung bestand daher darin, ein Verfahren zur Herstellung eines Katalysators, bestehend aus einem Trägerkörper und einer auf der Oberfläche des Trägerkörpers aufgebrachten katalytisch aktiven Oxidmasse, zur Verfügung zu stellen, mit dem Schalenkatalysatoren erhältlich sind, die als Schalenkatalysatoren mit verbesserter Aktivität und erhöhter Selektivität der Acroleinbildung für die gasphasenkatalytische Oxidation von Propen zu Acrolein geeignet sind.

Demgemäß wurde ein Verfahren zur Herstellung eines Katalysators, der aus einem Trägerkörper und einer auf der Oberfläche des Trägerkörpers aufgebrachten katalytisch aktiven Oxidmasse der allgemeinen Formel I

$$Mo_{12}Bi_aX^1_bFe_cX^2_dX^3_eO_y \qquad (I),$$

mit

$X^1 =$ Co und/oder Ni,
$X^2 =$ Si und/oder Al,
$X^3 =$ Li, Na, K, Cs und/oder Rb,
$0,2 \leq a \leq 1$,
$2 \leq b \leq 10$,
$0,5 \leq c \leq 10$,
$0 \leq d \leq 10$,
$0 \leq e \leq 0,5$ und
$y =$ eine Zahl, die unter der Voraussetzung der Ladungsneutralität durch die Wertigkeit und Häufigkeit der von Sauerstoff verschiedenen Elemente in I bestimmt wird,

besteht,
bei dem man aus Ausgangsverbindungen der elementaren Konstituenten der katalytisch aktiven Oxidmasse ein inniges Trockengemisch erzeugt, das innige Trockengemisch bei einer Temperatur von 150° bis 350°C, vorzugsweise 220 bis 280°C unter Erhalt einer Vorläufermasse thermisch behandelt, den Trägerkörper mit Wasser befeuchtet, danach durch Inkontaktbringen mit feinteiliger Vorläufermasse an der Oberfläche des befeuchteten Trägerkörpers eine Schicht der Vorläufermasse anheftet, anschließend den beschichteten Trägerkörper trocknet und abschließend den getrockneten, mit Vorläufermasse beschichteten, Trägerkörper bei einer Temperatur von 400 bis 600°C calciniert, gefunden, das dadurch gekennzeichnet ist, daß der Trägerkörper Ringgeometrie aufweist.

Die erfindungsgemäß zu verwendenden ringförmigen Trägerkörper sind vorzugsweise chemisch inert, d.h., sie greifen in den Ablauf der katalytischen Gasphasenoxidation des Propens zu Acrolein, die durch die erfindungsgemäß hergestellten Schalenkatalysatoren katalysiert wird, im wesentlichen nicht ein. Als Materialien für die Trägerkörper kommen erfindungsgemäß insbesondere Aluminiumoxid, Siliciumdioxid, Silicate wie Ton, Kaolin, Steatit, Bims, Aluminiumsilicat und Magnesiumsilicat, Siliciumcarbid, Zirkondioxid und Thoriumdioxid in Betracht.

Die Oberfläche des Trägerkörpers kann sowohl glatt als auch rauh sein. Mit Vorteil ist die Oberfläche des Trägerkörpers rauh, da eine erhöhte Oberflächenrauhigkeit in der Regel eine erhöhte Haftfestigkeit der aufgebrachten Schale an Katalysatorvorläufermasse bzw. an oxidischer Aktivmasse bedingt. Häufig liegt die Oberflächenrauhigkeit $R_z$ des Trägerkörpers im Bereich von 40 bis 200 µm, oft im Bereich von 40 bis 100 µm (bestimmt gemäß DIN 4768 Blatt 1 mit einem "Hommel Tester für DIN-ISO Oberflächenmeßgrößen" der Fa. Hommelwerke, DE).

Ferner kann das Trägermaterial porös oder unporös sein. Zweckmäßigerweise ist das Trägermaterial unporös (Gesamtvolumen der Poren auf das Volumen des Trägerkörpers bezogen $\leq$ 1 Vol.-%) .

Die Länge der erfindungsgemäß einzusetzenden Trägerringe beträgt in typischer Weise 2 bis 10 mm und ihr Außendurchmesser beträgt in typischer Weise 4 bis 10 mm. Die Wanddicke der Trägerringe liegt üblicherweise bei 1 bis 4 mm. Erfindungsgemäß geeignete Trägerringabmessungen sind auch 3 bis 6 mm (Länge), 4 bis 8 mm (Außendurchmesser) und 1 bis 2 mm (Wanddicke). Selbstverständlich kommt als erfindungsgemäße Ringgeometrie auch in Betracht 2 bis 4 mm (Länge), 4 bis 8 mm (Außendurchmesser) und 1 bis 2 mm (Wanddicke).

Erfindungsgemäß markante Trägerringgeometrien sind z.B. 7 mm x 3 mm x 1,5 mm (Außendurchmesser x Länge x Wanddicke) und 5 mm x 3 mm x 1,5 mm (Außendurchmesser x Länge x Wanddicke).

Die Dicke der auf den erfindungsgemäß erhältlichen Schalenkatalysatoren befindlichen aktiven Oxidmasse liegt in der Regel bei 10 bis 1000 µm. Sie kann aber auch 100 bis 700 µm, 200 bis 600 µm oder 300 bis 500 µm betragen. Mögliche Schalendicken sind auch 10 bis 500 µm oder 200 bis 300 µm.

Die Feinheit der auf die Oberfläche des ringförmigen Trägerkörpers aufzubringenden Vorläufermasse wird selbstredend an die gewünschte Dicke der aktiven Oxidmassenschale angepaßt. Für den Bereich einer Schalendicke von 100 bis 500 µm eignen sich z.B. Pulver aus Vorläufermasse, von denen wenigstens 50 % der Pulverpartikel ein Sieb der Maschenweite 1 bis 10 µm passieren und deren Anteil an Partikeln mit einer Längstausdehnung oberhalb von 50 µm weniger als 1 % (bezogen auf die Gesamtzahl der Partikel) beträgt. In der Regel entspricht die Verteilung der Längstausdehnungen der Pulverpartikel herstellungsbedingt einer Gaußverteilung.

Zur Erzielung der gewünschten Schalendicke ist es zweckmäßig, das erfindungsgemäße Verfahren periodisch zu wiederholen. D.h., der grundbeschichtete Trägerkörper bildet dann in der darauffolgenden Periode in erfindungsgemäßer Weise zunächst zu befeuchtenden und dann durch Kontakt mit trockener feinteiliger Vorläufermasse zu beschichtenden "Trägerkörper" usw.. Die Zugabe an feinteiliger Vorläufermasse und an Bindemittel erfolgen in der Regel kontinuierlich.

Für eine Durchführung des erfindungsgemäß anzuwendenden Beschichtungsverfahrens im technischen Maßstab empfiehlt sich daher z.B. das in der der DE-A 2909671 offenbarte Verfahrensprinzip.

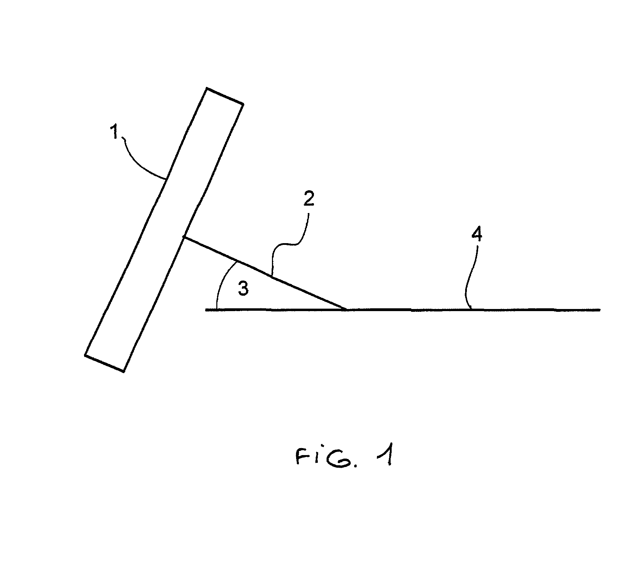

D.h., die zu beschichtenden Trägerkörper werden in zweckmäßiger Weise in einen geneigten (der Neigungswinkel (Winkel der Drehbehältermittelachse gegen die Horizontale; siehe Figur 1, in der 1 = Dragiertrommel (Seitenansicht), 2 = Trommelmittelachse, 3 = Neigungswinkel, 4 = Horizontale) beträgt $\geq 0°$ und $\leq 90°$, in der Regel $\geq 30°$ und $\leq 90°$) rotierenden Drehbehälter (z.B. Drehteller oder Dragiertrommel bzw. -kessel) vorgelegt.

Der rotierende Drehbehälter führt die Trägerkörper unter zwei in bestimmtem Abstand aufeineranderfolgend angeordneten Dosiervorrichtungen hindurch. Die erste der beiden Dosiervorrichtungen entspricht zweckmäßig einer Düse (z.B. eine mit Druckluft betriebenen Zerstäuberdüse), durch die die im rotierenden Drehteller rollenden Trägerkörper mit Wasser besprüht und kontrolliert befeuchtet werden. Die zweite Dosiervorrichtung befindet sich außerhalb des Zerstäubungskegels des als Bindemittel eingesprühten Wassers und dient dazu, die feinteilige Vorläufermasse zuzuführen (z.B. über eine Schüttelrinne oder Pulverschnecke). Die kontrolliert befeuchteten Trägerringe nehmen das zugeführte Vorläufermassenpulver auf, das sich durch die rollende Bewegung auf der äußeren Oberfläche der ringförmigen Trägerkörper zu einer zusammenhängenden Schale verdichtet (im inneren Kreis des hohlzylindrischen Trägerkörpers findet eine solche verdichtende Bewegung nicht statt, weshalb dieser im wesentlichen unbeschichtet bleibt).

Bei Bedarf durchläuft der so grundbeschichtete Trägerkörper im Verlauf der darauffolgenden Umdrehung wiederum die Sprühdüse, wird dabei kontrolliert befeuchtet, um im Verlauf der Weiterbewegung eine weitere Schicht feinteiliger Vorläufermasse aufnehmen zu können usw. (eine Zwischentrocknung ist in der Regel nicht erforderlich).

Die Entfernung des erfindungsgemäß als Wasser zu verwendenden Bindemittel kann z.B. durch Wärmezufuhr, z.B. durch Einwirkung von heißen Gasen, wie $N_2$ oder Luft, erfolgen. Selbredend kann die Trocknung auch in einem Trockenschrank erfolgen. Die Trocknungstemperatur beträgt in der Regel 100 bis 150°C.

Bemerkenswerterweise bewirkt das erfindungsgemäße Verfahren sowohl eine befriedigende Haftung von aufeinanderfolgenden Schichten aneinander, als auch der Grundschicht auf der Oberfläche des Trägerkörpers.

Wesentlich für die beschriebene Form des erfindungsgemäßen Verfahrens ist, daß die Befeuchtung der zu beschichtenden Oberfläche des Trägerkörpers in kontrollierter Weise vorgenommen wird. Kurz ausgedrückt heißt dies, daß man die Trägeroberfläche zweckmäßig so befeuchtet, daß diese zwar Wasser absorbiert aufweist, aber auf der Trägeroberfläche keine Flüssigphase als solche visuell in Erscheinung tritt. Ist die Trägerkörperoberfläche zu feucht, agglomeriert die feinteilige Vorläufermasse zu getrennten Agglomeraten, anstatt auf die Oberfläche aufzuziehen. Detaillierte Angaben hierzu finden sich in der DE-A 2909671.

Ein Vorzug des erfindungsgemäßen Verfahrens besteht darin, daß die Entfernung des als Bindemittel verwendeten Wassers in kontrollierter Weise z.B. durch Verdampfen vorgenommen werden kann. Im einfachen Fall kann dies durch Einwirkung heißer Gase entsprechender Temperatur (überlicherweise 100 bis 150°C) erfolgen. Durch Einwirkung heißer Gase kann aber auch nur eine Vortrocknung bewirkt werden. Die Endtrocknung kann dann beispielsweise in einem Trockenofen beliebiger Art (z.B. Bandtrockner) erfolgen.

Wesentliches Merkmal des erfindungsgemäßen Verfahrens ist, daß auf den Trägerkörper nicht die katalytisch aktive oxidische Masse als solche, sondern eine Vorläufermasse aufgetragen wird.

Der mit der Vorläufermasse beschichtete und getrocknete ringförmige Träger muß erfindungsgemäß zur Erzeugung der katalytisch aktiven oxidischen Masse calciniert werden. Dies kann innerhalb eines Zeitraums von wenigen Stunden (typische Calcinationsdauern sind 2 h bis 10 h, wobei die erforderliche Calcinationsdauer mit zunehmender Calcinationstemperatur abnimmt) bei Temperaturen von 400 bis 600°C, vorzugsweise bei Temperaturen von 430 bis 500°C erfolgen.

Die Calcination kann sowohl unter oxidierender, unter inerter oder unter reduzierender Atmosphäre erfolgen. Zweckmäßigerweise erfolgt sie unter Luft. Selbredend kann sie auch unter Vakuum durchgeführt werden. Zur Erzeugung einer Inertgasatmosphäre eignen sich z.B. inerte Gase wie molekularer Stickstoff und/oder Edelgase wie He, Ar. Die Durchführung der Calcination kann z.B. in einem Umluftofen erfolgen.

Zur Herstellung der Katalysatorvorläufermasse, mit der die ringförmigen Träger erfindungsgemäß zu beschichten sind, geht man üblicherweise von in an sich bekannter Weise geeigneten Quellen der Konstituenten der katalytisch aktiven, oxidischen Masse aus und erzeugt aus diesen ein möglichst inniges, in der Regel feinteiliges, Trockengemisch, welches dann bei einer Temperatur von 150 bis 350°C, vorzugsweise 220 bis 280°C, thermisch behandelt wird ( in der Regel 1 h bis 6 h). Diese thermische Behandlung kann ebenfalls unter inerter, reduzierender oder oxidierender Atmosphäre erfolgen. Üblicherweise erfolgt sie unter Luft. Die bei der thermischen Behandlung, unter der sich die verwendeten Quellen in der Regel zersetzen, entstehende Vorläufermasse kann dann in feinteiliger Form auf die Trägerringe aufgetragen werden.

Wesentlich ist, daß es sich bei den erfindungsgemäß zu verwendenden Quellen entweder bereits um Oxide handelt, oder um solche Verbindungen, die durch Erhitzen, wenigstens in Anwesenheit von Sauerstoff, in Oxide überführbar sind. Neben den Oxiden kommen daher als Ausgangsverbindungen vor allem Ammoniummetallate, Halogenide, Nitrate, Formiate, Oxalate, Acetate, Carbonate oder Hydroxide in Betracht.

Das innige Vermischen der Ausgangsverbindungen kann in trockener oder in nasser Form erfolgen. Erfolgt es in trockener Form, werden die Ausgangsverbindungen zweckmäßigerweise als feinteilige Pulver eingesetzt und nach Mischen sowie gegebenenfalls Verpressen erfindungsgemäß thermisch behandelt.

Vorzugsweise erfolgt das innige Vermische jedoch in nasser Form. Üblicherweise werden die Ausgangsverbindungen dabei in Form einer wäßrigen Lösung oder Suspension miteinander vermischt. Anschließend wird die wäßrige Masse getrocknet. Mit Vorteil erfolgt die Trocknung durch Sprühtrocknung (die Gaseintrittstemperatur beträgt dabei in der Regel 280 bis 420°C, und die Gasaustrittstemperatur liegt in typischer Weise bei 100 bis 150°C). Das bei der Sprühtrocknung anfallende Pulver erweiset sich für eine unmittelbare Weiterverarbeitung häufig als zu feinteilig. In diesen Fällen kann es unter Zusatz von Wasser geknetet werden. Im Anschluß an die Knetung wird das Knetgut zweckmäßig grob zerteilt und getrocknet (z.B. bei Temperaturen von 100 bis 150°C im Trockenschrank). An die Trocknung kann sich dann die erfindungsgemäß erforderliche thermische Behandlung bei 150 bis 350°C anschließen, bevor die erhaltene Vorläufermasse für den erfindungsgemäßen Beschichtungszweck z.B. durch Mahlen in die erforderliche feinteilige Form überführt wird.

Erfindungsgemäß bevorzugt sind solche Schalenkatalysatoren, deren katalytisch aktive Oxidmasse als $X^1$ nur Co aufweist. Bevorzugtes $X^2$ ist Si und $X^3$ ist vorzugsweise K, Na und/oder Cs, besonders bevorzugt ist $X^3$ = K.

Der stöchiometrische Koeffizient a beträgt mit Vorteil $0,4 \leq a \leq 1$, besonders bevorzugt $0,4 \leq a \leq 0,95$. Der stöchiometrische Koeffizient b liegt bevorzugt im Bereich $4 \leq b \leq 8$, und besonders bevorzugt im Bereich $6 \leq b \leq 8$. Der Wert für die Variable c liegt mit Vorteil im Bereich $1 \leq c \leq 5$ und mit besonderem Vorteil im Bereich $2 \leq c \leq 4$. Der stöchiometrische Koeffizient e ist zweckmäßigerweise > 0. Bevorzugt ist $0,01 \leq e \leq 0,5$ und besonders bevorzugt gilt $0,05 \leq e \leq 0,2$.

Der Wert für den stöchiometrischen Koeffizienten des Sauerstoff, y, ergibt sich aus der Wertigkeit und Häufigkeit der Kationen unter der Voraussetzung der Ladungsneuträlität. Günstig sind erfindungsgemäße Schalenkatalysatoren mit katalytisch aktiven Oxidmassen, deren molares Verhältnis von Co/Ni wenigstens 2:1, bevorzugt wenigstens 3:1 und besonders bevorzugt wenigstens 4:1 beträgt. Am besten liegt nur Co vor.

Bei besonders bevorzugten erfindungsgemäßen Schalenkatalysatoren liegt der Wert für 1,5 x (a + c) + b im Bereich $\geq 11$ und $\leq 14$, vorzugsweise im Bereich $\geq 11,5$ und $\leq 13$. Besonders bevorzugt sind Werte für 1,5 x (a + c) + b im Bereich $\geq 11,8$ und $\leq 12,5$.

Ferner sind erfindungsgemäß jene erfindungsgemäßen Schalenkatalysatoren geeignet, deren katalytisch aktive Oxidmasse einer in der DE-A 19855913 spezifizierten katalytisch aktiven Oxidmasse entspricht.

Die erfindungsgemäß erhältlichen Schalenkatalysatoren eigenen sich nicht nur für die selektive Gasphasenoxidation von Propen zu Acrolein, sondern auch für die partielle Gasphasenoxidation anderer organischer Verbindungen (anderer Alkene, Alkane, Alkanone oder Alkenole) zu α,β-ungesättigten Aldehyden und/oder Carbonsäuren. Beispielhaft genannt sei die Herstellung von Acrylsäure aus Acrolein und die Herstellung von Methacrolein und Methacrylsäure aus iso-Buten, iso-Butan, tert.-Butanol oder tert.-Butylmethylether.

Die unter Anwendung der erfindungsgemäßen Schalenkatalysatoren für die gasphasenkatalytische Oxidation von Propen zu Acrolein einzuhaltenden allgemeinen Reaktionsbedingungen finden sich z.B. in der DE-A 4023239 sowie in der DE-A 4431957.

Vor allem eigenen sich die erfindungsgemäßen Schalenkatalysatoren zur Durchführung der partiellen Gasphasenoxidation von Propen zu Acrolein unter Anwendung erhöhter Propenbelastungen der Katalysatorbeschickung, wie es z.B. in der DE-A 19955168, in der DE-A 19948523 und in der DE-A 19948248 beschrieben ist. Wesentlich ist, daß sie auch bei erhöhter Propenbelastung eine erhöhte Aktivität sowie eine erhöhte Selektivität der Acroleinbildung aufweisen.

Abschließend sei festgehalten, daß die im Rahmen einer gasphasenkatalytisch oxidativen Propenoxidation zu Acrolein verbrauchten, erfindungsgemäß erhältlichen Schalenkatalysatoren wie in der EP-A 339119 beschrieben regeneriert werden können.

Beispiele und Vergleichsbeispiele

A) Herstellung einer Vorläufermasse A der Stöchiometrie $Mo_{12}Bi_{0,6}Fe_3Co_7Si_{1,6}K_{0,08}O_x$

Lösung A:

Zu 3530,05 g einer auf 60°C erwärmten wäßrigen Cobalt(II)-nitrat-Lösung (12,4 Gew.-% Co) wurden über einen Pulvertrichter unter Rühren innerhalb einer Minute unter Aufrechterhaltung der 60°C 1252,51 g Eisen(III)-nitrat (14,2 Gew.-% Fe) zugegeben. Nach Beendigung der Zugabe wurde 30 Minuten bei 60°C nachgerührt. Abschließend wurden unter Beibehaltung der 60°C über einen Tropftrichter innerhalb von zwei Minuten 1198,99 g einer wäßrigen Bismutnitratlösung (11,1 Gew.-% Bi) eingerührt. Nach zehn minütigem Nachrühren bei 60°C wurde eine klare, rotgefärbte wäßrige Lösung A erhalten.

Lösung B:

In 2500 g Wasser wurden 10,18 g einer wäßrigen KOH-Lösung (46,8 Gew.-% KOH) eingerührt. Anschließend wurde die Lösung unter Rühren auf 60°C aufgeheizt. Dann gab man unter Aufrechterhaltung der 60°C unter Rühren portionsweise 2249,72 g Ammoniumheptamolybdat hinzu und rührte noch eine Stunde bei 60°C nach. Es wurde eine leicht getrübte blaßgelbe wäßrige Lösung B erhalten.

Fällung:

Zur 60°C aufweisenden wäßrigen Lösung B wurde mittels einer Pumpe unter Rühren innerhalb von 15 min die 60°C aufweisende wäßrige Lösung A gegeben. Nach Beendigung der Zugabe wurde noch 5 min bei 60°C nachgerührt. Dann wurden unter Rühren 204,11 g Kieselsol (Ludox®TM, Du Pont, 50 Gew.-% $SiO_2$, Dichte: 1,39 g/ml, pH: 8,8, Alkaligehalt ≤ 0,5 Gew.-%) zugegeben und weitere fünf Minuten bei 60°C nachgerührt.

Sprühtrocknung:

Das erhaltene wäßrige Gemisch wurde in einem Sprühtrockner der Firma Niro (Sprühtrockner Niro A/S Atomizer Transportable Minor Anlage, Zentrifugalzerstäuber der Fa. Niro, DK) sprühgetrocknet. Die Vorlagetemperatur betrug 60°C. Die Gaseintrittstemperatur betrug 360 ± 10°C, die Gasaustrittstemperatur betrug 115 ± 5°C. Das gesamte wäßrige Gemisch wurde mit einer Förderleistung von 2 l/h über eine Zweistoffdüse mit aufgesetztem Zerstäuberrad mit einem Sprühdüsenvordruck von 5,2 bar mit Luft als Trägergas (40m³/h) im Gleichstrom versprüht. Nach der Pulverabscheidung in einem Zyklon wurde ein Sprühpulver mit einer Partikalgröße von 20 bis 25 μm erhalten.

Knetung:

400 g des Sprühpulvers wurden in einem 1 1-Kneter der Firma Werner & Pfleiderer vom Typ LUK 075 unter Zugabe von 150 ml Wasser geknetet. Der Kneter verfügte über zwei gegenläufig betriebene Sigmaschaufeln. Die Knetung erfolgte in drei Schritten, die 5, 10 und 15 Minuten dauerten. Vor dem dritten Knetschritt wurde das Knetgut von Hand zerteilt, durchmischt und von den Kentschaufeln abgelöst um eine gleichmäßige Vermischung zu gewährleisten.

Trocknung:

Im Anschluß an die Knetung wurde das Knetgut grob zerteilt und während 2 Stunden in einem Trockenschrank der Firma Binder, DE, vom Typ FD 53 (53 l Innenvolumen) bei 120°C getrocknet.

Zersetzung:

In einem Umluftofen der Firma Nabertherm, DE, vom Typ N60/A (60 l Innenvolumen) wurde das getrocknete Knetgut thermisch behandelt. Der Ofen wurde zunächst innerhalb von 2 Stunden auf 240°C erwärmt und 10 min bei dieser Temperatur gehalten. Anschließend wurde innerhalb von 60 min. auf 280°C erwärmt. Diese Temperatur wurde für eine Stunde konstant gehalten. Während des gesamten Prozesses wurde durch den Umluftofen ein Gasstrom von 300 Nl/l Luft geleitet. Erhalten wurde so eine Vorläufermasse A.

B) Herstellung einer Vorläufermasse B der Stöchiometrie B $Mo_{12}Bi_{0,2}Fe_{4,0}Co_7Si_{1,6}K_{0,08}O_x$

Lösung A':

Zu 3547,38 g einer auf 60°C erwärmten wäßrigen Cobalt(II)-nitrat-Lösung (12,4 Gew.-% Co) wurden über einen Pulvertrichter unter Rühren und unter Aufrechterhaltung der 60°C 1678,22 g Eisen(III)-nitrat(14,2 Gew.-% Fe) innerhalb einer Minute zugegeben. Nach Beendigung der Zugabe wurde 30 Minuten bei 60°C nachgerührt. Abschließend erfolgte innerhalb von 2 min. die Zugabe von 401,63 g einer wäßrigen Bismutnitratlösung (11,1 Gew.-% Bi) über einen Tropftrichter und unter Rühren bei 60°C. Danach wurde noch 10 min bei 60°C nachgerührt. Es wurde eine klare rotgefärbte Lösung A' erhalten.

Lösung B':

In 2500 g Wasser wurden 10,23 g einer wäßrigen KOH-Lösung (46,8 Gew.-% KOH) eingerührt. Die wäßrige Lösung wurde unter Rühren auf 60°C erwärmt. Anschließend wurden unter Aufrechterhaltung der 60°C unter Rühren portionsweise 2260,76 g Ammoniumheptamolybdat hinzugegeben. Danach wurde noch 1 Stunde bei 60°C gerührt und so eine leicht getrübte blaßgelbe Lösung B' erhalten.

Fällung:

Zur vorgelegten, 60°C aufweisenden wäßrigen Lösung B' wurde die 60°C aufweisende Lösung A' innerhalb von 15 min. unter Rühren über eine Pumpe zugegeben. Nach Beendigung der Zugabe wurde noch 5 min bei 60°C nachgerührt. Anschließend wurden unter Aufrechterhaltung der 60°C 205,11 g Kieselsol (Ludox-®TM, Du Pont, 50 Gew.-% $SiO_2$, Dichte: 1,39 g/ml, pH:8,8, Alkaligehalt ≤ 0,5 Gew.-%) zugegeben und weitere fünf Minuten bei 60°C nachgerührt.
Sprühtrocknung, Knetung, Trocknung und Zersetzung wurden anschließend wie unter A) durchgeführt und so eine Vorläufermasse B erhalten.

C) Herstellung von erfindungsgemäßen Schalenkatalysatoren und Vergleichsschalenkatalysatoren

Allgemeine Herstellmerkmale:

Die für die Beschichtung einzusetzende Vorläufermasse wurde in einer Zentrifugalmühle (Typ ZM 100, Fa. Retsch, DE) auf eine Korngröße > 0 und ≤ 0,12 mm gemahlen. Im einzelnen ist die Korngrößenverteilung wie folgt beschaffen:

| D (µm) | 1 | 1,5 | 2 | 3 | 4 | 6 | 8 | 12 | 16 | 24 | 32 |
|---|---|---|---|---|---|---|---|---|---|---|---|
| x | 80,5 | 76,3 | 67,1 | 53,4 | 41,6 | 31,7 | 23 | 13,1 | 10,8 | 7,7 | 4 |
| y | 19,5 | 23,7 | 32,9 | 46,6 | 58,4 | 68,3 | 77 | 86,9 | 89,2 | 92,3 | 96 |

| D (µm) | 48 | 64 | 96 | 128 |
|---|---|---|---|---|
| x | 2,1 | 2 | 0 | 0 |
| y | 97,9 | 98 | 100 | 100 |

Dabei sind:

D = Durchmesser des Korns.
X = der prozentuale Anteil der Körner, deren Durchmesser ≥ D ist.
Y = der prozentuale Anteil der Körner, deren Durchmesser < D ist.

124 g der gemahlenen Vorläufermasse wurden auf 250 g Trägerkörper ($R_z$ = 45 µm, Trägermaterial = Steatit, Porengesamtvolumen des Trägers ≤ 1 Vol.-% (bezogen auf Trägergesamtvolumen)) aufgebracht. Dazu wurde der Träger in einer Dragiertrommel (2 1 Innenvolumen, Neigungswinkel der Trommelmittelachse gegen die Horizontale = 30°) vorgelegt. Die Trommel wurde mit 25 Umdrehungen je Minute in Rotation versetzt. Über eine mit Druckluft betriebene Zerstäuberdüse wurden über 60 min. hinweg ca. 45 ml flüssiges Bindemittel auf den Träger gesprüht. Die Düse war dabei derart installiert, daß der Sprühkegel die in der Trommel durch Mitnahmebleche an den obersten Punkt der geneigten Trommel beförderten Trägerkörper in der oberen Hälfte der Abrollstrecke benetzte. Die feinteilige Vorläufermasse wurde über eine Pulverschnecke in die Trommel eingetragen, wobei der Punkt der Pulverzugabe innerhalb der Abrollstrecke aber unterhalb des Sprühkegels lag. Durch die periodische Wiederholung von Benetzung und Pulveraufdosierung wurde der grundbeschichtete Trägerkörper in der darauffolgenden Periode selbst zum Trägerkörper. Nach Abschluß der Beschichtung wurde der Beschichtete Trägerkörper während 2 Stunden bei 120°C im Trockenschrank (Firma Binder, DE, Innenvolumemn 53 l) getrocknet. Anschließend wurden die getrockneten Schalenkatalysatorvorläufer in einem von 800 Nl/h Luft durchströmten Umluftofen der Firma Heraeus ,DE (Typ K 750/2 S, Innenvolumen 55 l) calciniert.

Dazu wurde der Umluftofen innerhalb von 210 min. linear von 25°C auf 470°C erwärmt. Diese Temperatur wurde dann während 6 h aufrechterhalten. Die auf diese Art und Weise hergestellten Schalenkatalysatoren wiesen in allen Fällen eine Aktivmassenschichtdicke von 370 ± 30 µm auf.

Vergleichsschalenkatalysator VS1:  Verwendet wurde die Vorläufermasse A, als Trägerkörper wurden Kugeln mit einem Durchmesser von 2,5 bis 3,5 mm verwendet, das flüssige Bindemittel war Wasser;

erfindungsgemäßer Schalenkatalysator S1:  wie VS1, als Trägerkörper wurden jedoch Hohlzylinder mit einem Außendurchmesser von 7 mm, einer Länge von 3 mm und einem Innendurchmesser von 4 mm verwendet;

Vergleichsschalenkatalysator VS2:  Wie S1, als flüssiges Bindemittel wurde jedoch eine 25 gew.-%ige Lösung von Glycerin in Wasser verwendet;

Vergleichsschalenkatalysator VS3:  Wie S1, zur Beschichtung wurde jedoch nicht die Vorläufermasse A, sondern eine 6 h bei 470°C im oben genannten Umluftofen (der von 800 Nl/h Luft durchströmt war) der Firma Heraeus calcinierte Vorläufermasse A verwendet (die Erwärmung des Umluftofen von 25°C auf 470°C erfolgte innerhalb von 210 min. durch lineare Temperaturerhöhung). Die nach der Beschichtung der Trägerkörper und Trocknung anstehende Calcinierung wurde unterlassen.

Vergleichsschalenkatalysator VS4:  Wie VS1, als Vorläufermasse wurde jedoch die Vorläufermasse B verwendet;

Erfindungsgemäßer Schalenkatalysator S2:  Wie S1, als Vorläufermasse wurde jedoch die Vorläufermasse B verwendet;

Vergleichsschalenkatalysator VS5:  Wie VS2, als Vorläufermasse wurde jedoch die Vorläufermasse B verwendet.

D) Testung der unter C hergestellten Schalenkatalysatoren

Mit den Schalenkatalysatoren wurde jeweils ein Reaktionsrohr aus V2A Stahl (Außendurchmesser = 21 mm, Innendurchmesser = 15 mm) beschickt. Die Beschickungslänge wurde in allen Fällen so gewählt, daß das Katalysatorfestbett 32 bis 34 g Aktivmasse enthielt.

Das Reaktionsrohr wurde auf seiner gesamten Länge mit einem es umfließenden Salzbad temperiert. Als Reaktionsgasausgangsgemisch wurde ein Gemisch aus 5 Vol.-% Propen, 9,5 Vol.-% Sauerstoff und 85,5 Vol.-% Stickstoff verwendet. Die Belastung des Reaktionsrohres mit Reaktionsgasausgangsgemisch betrug in allen Fällen 100Nl/h. Die Salzbadtemperatur wurde in allen Fällen so eingestellt, daß bei einmaligem Durchgang durch das Reaktionsrohr ein Propenumsatz U von 95 mol-% erzielt wurde.

Im Produktgasstrom wurde durch gaschromatographische Analyse die Selektivität S der Wertproduktbildung an Acrolein ermittelt.

Dabei sind U und S wie folgt definiert:

$$U(\text{mol.-\%}) = \frac{\text{Molzahl Propen im Ausgangsgemisch - Molzahl Propen im Produktgemisch}}{\text{Molzahl Propen im Ausgangsgamisch}} \times 100$$

$$S(\text{mol.-\%}) = \frac{\text{Molzahl Acrolein im Produktgemisch}}{\text{Molzahl Propen im Ausgangsgemisch - Molzahl Propen im Produktgemisch}} \times 100$$

Die nachfolgende Tabelle zeigt die mit den unterschiedlichen Schalenkatalysatoren erforderlichen Salzbadtemperaturen $T_s[°C]$ (je niedriger die benötigte Salzbadtemperatur, desto höher die Katalysatoraktivität) sowie die erzielten Selektivitäten der Acroleinbildung und die genauen Umsätze des Propens. Die Selektivitätsangaben sind dabei relative Selektivitätsangaben $S_R$. D.h., innerhalb der Reihe von Schalenkatalysatoren VS1, S1, VS2 und VS3 wurde die mit VS1 erzielte Selektivität der Acroleinbildung gleich 100 gesetzt und die mit den anderen drei Schalenkatalysatoren erzielte Selektivität der Acroleinbildung darauf bezogen.

In entsprechender Weise wurde innerhalb der Reihe von Schälenkatalysatoren VS4, S2 und VS5 die mit VS4 erzielte Selektivität der Acroleinbildung gleich 100 gesetzt.

Tabelle

| Schalenkatalysator | $T_s$ [°C] | U[mol-%] | $S_R$ |
|---|---|---|---|
| VS1 | 363 | 94,9 | 100 |
| S1 | 335 | 95,2 | 102,3 |
| VS2 | 347 | 94,2 | 101,9 |
| VS3 | 370 | 94,8 | 98,7 |
| VS4 | 359 | 95,0 | 100 |
| S2 | 334 | 95,1 | 102,1 |
| VS5 | 345 | 94,9 | 100,4 |

**Patentansprüche**

**1.** Verfahren zur Herstellung eines Katalysators, der aus einem Trägerkörper und einer auf der Oberfläche des Trägerkörpers aufgebrachten katalytisch aktiven Oxidmasse der allgemeinen Formel I

$$\text{Mo}_{12}\text{Bi}_a\text{X}^1_b\text{Fe}_c\text{X}^2_d\text{X}^3_e\text{O}_y \tag{I},$$

mit

$X^1$= Co und/oder Ni,
$X^2$= Si und/oder Al,
$X^3$= Li, Na, K, Cs und/oder Rb,
$0{,}2 \leq a \leq 1$,
$2 \leq b \leq 10$,
$0{,}5 \leq c \leq 10$,
$0 \leq d \leq 10$,
$0 \leq e \leq 0{,}5$ und
y= eine Zahl, die unter der Voraussetzung der Ladungsneutralität durch die Wertigkeit und Häufigkeit der von Sauerstoff verschiedenen Elemente in I bestimmt wird,

besteht,
bei dem man aus Ausgangsverbindungen der elementaren Konstituenten der katalytisch aktiven Oxidmasse ein inniges Trockengemisch erzeugt, das innige Trockengemisch bei einer Temperatur von 150 bis 350°C unter Erhalt einer Vorläufermasse thermisch behandelt, den Trägerkörper mit Wasser befeuchtet, danach durch Inkontaktbringen mit feinteiliger Vorläufermasse an der Oberfläche des befeuchteten Trägerkörpers eine Schicht der Vorläufermasse anheftet, anschließend den beschichteten Trägerkörper trocknet und abschließend den getrockneten, mit Vorläufermasse beschichteten, Trägerkörper bei einer Temperatur von 400 bis 600°C calciniert, **dadurch gekennzeichnet, daß** der Trägerkörper Ringgeometrie aufweist.

2.  Verfahren nach Anspruch 1, **dadurch gekennzeichnet, daß** der Trägerkörper aus Aluminiumoxid, Siliciumdioxid, Ton, Kaolin, Steatit, Bims, Aluminiumsilicat, Magnesiumsilicat, Siliciumcarbid, Zirkoniumdioxid oder Thoriumdioxid besteht.

3.  Verfahren nach Anspruch 1 oder 2, **dadurch gekennzeichnet, daß** die Oberflächenrauhigkeit $R_z$ des Trägerkörpers 40 bis 200 µm beträgt.

4.  Verfahren nach einem der Ansprüche 1 bis 3, **dadurch gekennzeichnet, daß** das Gesamtvolumen der Poren des Trägerkörpers auf das Volumen des Trägerkörpers bezogen ≤ 1 Vol.-% beträgt.

5.  Verfahren nach einem der Ansprüche 1 bis 4, **dadurch gekennzeichnet, daß** die Ringe eine Länge von 2 bis 10 mm, einen Außendurchmesser von 4 bis 10 mm und eine Wanddicke von 1 bis 4 mm aufweisen.

6.  Verfahren nach einem der Ansprüche 1 bis 4, **dadurch gekennzeichnet, daß** die Ringe eine Länge von 3 bis 6 mm, einen Außendurchmesser von 4 bis 8 mm und eine Wanddicke von 1 bis 2 mm aufweisen.

7.  Verfahren nach einem der Ansprüche 1 bis 4, **dadurch gekennzeichnet, daß** die Ringe eine Länge von 2 bis 4 mm, einen Außendurchmesser von 4 bis 8 mm und eine Wanddicke von 1 bis 2 mm aufweisen.

8.  Verfahren nach einem der Ansprüche 1 bis 7, **dadurch gekennzeichnet, daß** die Schichtdicke der auf die Oberfläche des Trägerkörpers aufgebrachten katalytisch aktiven Oxidmasse 10 bis 1000 µm beträgt.

9.  Verfahren nach einem der Ansprüche 1 bis 7, **dadurch gekennzeichnet, daß** die Schichtdicke der auf die Oberfläche des Trägerkörpers aufgebrachten katalytisch aktiven Oxidmasse 100 bis 500 µm beträgt.

10. Verfahren zur Herstellung eines Katalysators gemäß einem der Ansprüche 1 bis 8, **dadurch gekennzeichnet, daß** das Verfahren gemäß einem der Ansprüche 1 bis 8 in periodischer Weise wiederholt angewendet wird, d.h., der grundbeschichtete Trägerkörper bildet in der darauf folgenden Periode zu befeuchtenden und dann durch Kontakt mit feinteiliger Vorläufermasse zu beschichtenden "Trägerkörper" usw..

11. Verfahren nach Anspruch 10, **dadurch gekennzeichnet, daß** die zu beschichtenden Trägerkörper in einen rotierenden Drehbehälter gefüllt werden, der die Trägerkörper periodisch unter zwei aufeinanderfolgend angeordneten Dosiervorrichtungen hindurchführt, von denen die erste die im rotierenden Drehbehälter rollenden Trägerkörper durch Besprühen mit Wasser befeuchtet und die zweite die Vorläufermasse in feinteiliger Form zudosiert.

12. Verfahren nach einem der Ansprüche 1 bis 11, **dadurch gekennzeichnet, daß** $X^1$ ausschließlich Co ist.

13. Verfahren nach einem der Ansprüche 1 bis 12, **dadurch gekennzeichnet, daß** $X^2$ ausschließlich Si ist.

14. Verfahren nach einem der Ansprüche 1 bis 13, **dadurch gekennzeichnet, daß** $X^3$ ausschließlich K ist.

15. Katalysator, erhältlich nach einem Verfahren gemäß einem der Ansprüche 1 bis 14.

16. Verfahren der gasphasenkatalytischen Oxidation von Propen zu Acrolein, **dadurch gekennzeichnet, daß** als Katalysator ein solcher gemäß Anspruch 15 verwendet wird.

17. Verwendung eines Katalysators gemäß Anspruch 15 zur gasphasenkatalytischen Oxidation von Propen zu Acrolein.

**Claims**

1.  A process for producing a catalyst comprising a support body and a catalytically active oxide composition of the formula I

$$Mo_{12}Bi_aX^1_bFe_cX^2_dX^3_eO_y \qquad (I),$$

where

$X^1$ = Co and/or Ni,
$X^2$ = Si and/or Al,
$X^3$ = Li, Na, K, Cs and/or Rb,
$0.2 \leq a \leq 1$,
$2 \leq b \leq 10$,
$0.5 \leq c \leq 10$,
$0 \leq d \leq 10$,
$0 \leq e \leq 0.5$ and
y= a number required to achieve electrical neutrality and determined by the valence and abundance of the elements other than oxygen in I,

applied to the surface of the support body,
in which an intimate dry mixture is produced from starting compounds of the elemental constituents of the catalytically active oxide composition, the intimate dry mixture is treated thermally at from 150 to 350°C to give a precursor composition, the support body is moistened with water, a layer of the precursor composition is then applied to the surface of the moistened support body by bringing it into contact with finely divided precursor composition, the coated support body is subsequently dried and the dried support body coated with precursor composition is finally calcined at from 400 to 600°C, wherein the support body has the geometry of a ring.

2. A process as claimed in claim 1, wherein the support body comprises aluminium oxide, silicon dioxide, clay, kaolin, steatite, pumice, aluminum silicate, magnesium silicate, silicon carbide, zirconium dioxide or thorium dioxide.

3. A process as claimed in claim 1 or 2, wherein the surface roughness $R_z$ of the support body is from 40 to 200 $\mu$m.

4. A process as claimed in any of claims 1 to 3, wherein the total volume of the pores of the support body is $\leq$ 1% of the volume of the support body.

5. A process as claimed in any of claims 1 to 4, wherein the rings have a length of from 2 to 10 mm, an external diameter of from 4 to 10 mm and a wall thickness of from 1 to 4 mm.

6. A process as claimed in any of claims 1 to 4, wherein the rings have a length of from 3 to 6 mm, an external diameter of from 4 to 8 mm and a wall thickness of from 1 to 2 mm.

7. A process as claimed in any of claims 1 to 4, wherein the rings have a length of from 2 to 4 mm, an external diameter of from 4 to 8 mm and a wall thickness of from 1 to 2 mm.

8. A process as claimed in any of claims 1 to 7, wherein the layer thickness of the catalytically active oxide composition applied to the surface of the support body is from 10 to 1000 $\mu$m.

9. A process as claimed in any of claims 1 to 7, wherein the layer thickness of the catalytically active oxide composition applied to the surface of the support body is from 100 to 500 $\mu$m.

10. A process for producing a catalyst as claimed in any of claims 1 to 8, wherein the process as claimed in any of claims 1 to 8 is repeated periodically, i.e. the initially coated support body forms the "support body" to be moistened and then coated by contact with finely divided precursor composition in the next cycle, and so forth.

11. A process as claimed in claim 10, wherein the support bodies to be coated are placed in a rotating vessel which conveys the support bodies periodically under two successive metering devices of which the first moistens the support bodies rolling in the rotating vessel by spraying with water and the second meters in the precursor composition in finely divided form.

12. A process as claimed in any of claims 1 to 11, wherein $X^1$ is exclusively Co.

**13.** A process as claimed in any of claims 1 to 12, wherein $X^2$ is exclusively Si.

**14.** A process as claimed in any of claims 1 to 13, wherein $X^3$ is exclusively K.

**15.** A catalyst obtainable by a process as claimed in any of claims 1 to 14.

**16.** A process for the catalytic gas-phase oxidation of propene to acrolein, wherein the catalyst used is a catalyst as claimed in claim 15.

**17.** The use of a catalyst as claimed in claim 15 for the catalytic gas-phase oxidation of propene to acrolein.

**Revendications**

**1.** Procédé de préparation d'un catalyseur qui est constitué d'un corps de support et d'une masse d'oxyde catalytiquement active appliquée sur la surface du corps de support et répondant à la formule générale (I) :

$$Mo_{12}Bi_aX^1{}_bFe_cX^2{}_dX^3{}_eO_y \qquad (I),$$

où

X$^1$ = Co et/ou Ni,
X$^2$ = Si et/ou Al,
X$^3$ = Li, Na, K, Cs et/ou Rb,
$0,2 \leq a \leq 1$,
$2 \leq b \leq 10$,
$0,5 \leq c \leq 10$,
$0 \leq d \leq 10$,
$0 \leq e \leq 0,5$, et
y = un chiffre qui, dans la condition de la neutralité de la charge, est déterminé par la valence et la fréquence des éléments différents de l'oxygène dans I,

dans lequel, à partir des composés de départ des constituants élémentaires de la masse d'oxyde catalytiquement active, on produit un mélange sec intime, on traite thermiquement le mélange sec intime à une température de 150 à 350°C avec obtention d'une masse de précurseurs, on humidifie le corps de support avec de l'eau, puis, par mise en contact d'une masse de précurseurs finement divisée sur la surface du corps de support humidifié, on fait adhérer une couche de la masse de précurseurs, puis on sèche le corps de support revêtu et ensuite on calcine le corps de support revêtu d'une masse de précurseurs, séché, à une température de 400 à 600°C, **caractérisé en ce que** le corps de support présente une géométrie annulaire.

**2.** Procédé suivant la revendication 1, **caractérisé en ce que** le corps de support est constitué d'oxyde d'aluminium, de dioxyde de silicium, d'argile, de kaolin, de stéatite, de pierre ponce, de silicate d'aluminium, de silicate de magnésium, de carbure de silicium, de dioxyde de zirconium ou de dioxyde de thorium.

**3.** Procédé suivant l'une des revendications 1 et 2, **caractérisé en ce que** la rugosité de surface $R_z$ du corps de support est de 40 à 200 μm.

**4.** Procédé suivant l'une des revendications 1 à 3, **caractérisé en ce que** le volume total des pores du corps de support par rapport au volume du corps de support est ≤ 1 % en volume.

**5.** Procédé suivant l'une des revendications 1 à 4, **caractérisé en ce que** les anneaux présentent une longueur de 2 à 10 mm, un diamètre externe de 4 à 10 mm et une épaisseur de paroi de 1 à 4 mm.

**6.** Procédé suivant l'une des revendications 1 à 4, **caractérisé en ce que** les anneaux présentent une longueur de 3 à 6 mm, un diamètre externe de 4 à 8 mm et une épaisseur de paroi de 1 à 2 mm.

**7.** Procédé suivant l'une des revendications 1 à 4, **caractérisé en ce que** les anneaux présentent une longueur de 2 à 4 mm, un diamètre externe de 4 à 8 mm et une épaisseur de paroi de 1 à 2 mm.

**8.** Procédé suivant l'une des revendications 1 à 7, **caractérisé en ce que** l'épaisseur de couche de la masse d'oxydes catalytiquement active, appliquée sur la surface du corps de support, est de 10 à 1.000 $\mu$m.

**9.** Procédé suivant l'une des revendications 1 à 7, **caractérisé en ce que** l'épaisseur de couche de la masse d'oxydes catalytiquement active, appliquée sur la surface du corps de support, est de 100 à 500 $\mu$m.

**10.** Procédé de préparation d'un catalyseur suivant l'une des revendications 1 à 8, **caractérisé en ce que** le procédé suivant l'une des revendications 1 à 8 est appliqué de façon répétée de manière périodique, c'est-à-dire que le corps de support revêtu de base forme dans la période suivante le "corps de support" à humidifier et ensuite, par contact avec une masse de précurseurs finement divisée, le "corps de support" à revêtir, etc.

**11.** Procédé suivant la revendication 10, **caractérisé en ce que** le corps de support à revêtir est chargé dans un récipient rotatif que le corps de support traverse de manière périodique avec deux dispositifs de dosage agencés successivement, dont le premier humidifie par aspersion le corps de support roulant dans le récipient rotatif et le deuxième ajoute la masse de précurseurs sous une forme finement divisée.

**12.** Procédé suivant l'une des revendications 1 à 11, **caractérisé en ce que** $X^1$ est exclusivement Co.

**13.** Procédé suivant l'une des revendications 1 à 12, **caractérisé en ce que** $X^2$ est exclusivement Si.

**14.** Procédé suivant l'une des revendications 1 à 13, **caractérisé en ce que** $X^3$ est exclusivement K.

**15.** Catalyseur que l'on peut obtenir suivant un procédé selon l'une des revendications 1 à 14.

**16.** Procédé d'oxydation catalytique en phase gazeuse de propène avec formation d'acroléine, **caractérisé en ce que**, comme catalyseur, on utilise un catalyseur suivant la revendications 15.

**17.** Utilisation d'un catalyseur suivant la revendication 15, pour l'oxydation catalytique en phase gazeuse de propène pour former de l'acroléine.

FIG. 1